# EUROPÄISCHE PATENTSCHRIFT

(11) **EP 1 243 323 B1**
(45) Veröffentlichungstag und Bekanntmachung des Hinweises auf die Patenterteilung: **05.01.2005**
(21) Anmeldenummer: 01107158.6
(22) Anmeldetag: 22.03.2001
(51) Int. Cl.: B01J 13/02

(54) **Nanokapseln**
Nanocapsules
Nanocapsules

(43) Veröffentlichungstag der Anmeldung: 25.09.2002
(73) Patentinhaber: Cognis Iberia, S.L., 08755 Castellbisbal, (Barcelona) (ES)
(72) Erfinder: Tacies, Ana, Barcelona (ES); Copete Vidal, Teresa, 08190 Sant Cugat del Vallès, Barcelona (ES); Pi Subirana, Rafael, Dr., 08400 Granollers, (Barcelona) (ES); Viladot Petit, Josep-Lluis, 08018 Barcelona (ES)
(74) Vertreter: Fabry, Bernd, Dr.

(56) Entgegenhaltungen:
- EP-A- 1 064 910
- EP-A- 1 064 913
- EP-A- 1 077 060
- DATABASE WPI Section Ch, Week 198847 Derwent Publications Ltd., London, GB; Class B05, AN 1988-333837 XP002174165 & JP 63 246333 A (TOYO CAPSULE KK), 13. Oktober 1988 (1988-10-13)

## Beschreibung

### Gebiet der Erfindung

Die Erfindung befindet sich auf den Gebieten der Kosmetik bzw. Pharmazie sowie der Textilausrüstungsmittel und betrifft neue, besonders feinteilige Mikrokapseln ("Nanokapseln"), ein Verfahren zu deren Herstellung, deren Verwendung in der Kosmetik und Textiltechnik sowie kosmetische bzw. pharmazeutische Zubereitungen sowie Textilien und Fasern, die diese Nanokapseln enthalten.

### Stand der Technik

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt. Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].** Man unterscheidet dabei im wesentlichen die beiden folgenden Verfahren:
(1) Mikrokapseln mit mittleren Durchmessern im Bereich von 0,1 bis 5 mm, bestehend aus einer Hüllmembran und einer mindestens ein Wachs enthaltenden Matrix, erhältlich, indem man
   (a) aus Gelbildnern, Chitosanen und Wirkstoffen eine Matrix zubereitet,
   (b) gegebenenfalls die Matrix in einer Ölphase dispergiert,
   (c) die dispergierte Matrix mit wässrigen Lösungen anionischer Polymere behandelt und gegebenenfalls dabei die Ölphase entfernt.
(2) Mikrokapseln mit mittleren Durchmessern im Bereich von 0,1 bis 5 mm, bestehend aus einer Hüllmembran und einer mindestens ein Wachs enthaltenden Matrix, erhältlich, indem man
   (a) aus Gelbildnern, anionischen Polymeren und Wirkstoffen eine Matrix zubereitet,
   (b) gegebenenfalls die Matrix in einer Ölphase dispergiert,
   (c) die dispergierte Matrix mit wässrigen Chitosanlösungen behandelt und gegebenenfalls dabei die Ölphase entfernt.

Die Mikrokapseln des Stands der Technik sind jedoch nicht in jeder Hinsicht zufriedenstellend. So besteht der Wunsch, die verkapselten Wirkstoffe zielgenauer einsetzen zu können und insbesondere die Beständigkeit zu verbessern.

Die Aufgabe der vorliegenden Erfindung hat somit darin bestanden, einen Weg aufzuzeigen, wie man die Leistungsfähigkeit verkapselter Wirkstoffe am Ort, an dem sie ihre Eigenschaften entfallen sollen, verbessern kann. Gleichzeitig sollten die Wasserresistenz sowie die Beständigkeit der Kapseln gegenüber Wärmebelastung deutlich verbessert werden.

### Beschreibung der Erfindung

Gegenstand der Erfindung sind neue Nanokapseln mit mittleren Durchmessern im Bereich von 10 bis 5000, vorzugsweise 50 bis 1000 und insbesondere 100 bis 500 nm, bestehend aus einer Hüllmembran und einer mindestens einen Wirkstoff enthaltenden Matrix, die man erhält, indem man
(a) aus Lecithinen bzw. Phospholipiden, Wachskörpern und Wirkstoffen eine Matrix herstellt,
(b) diese mit wässrigen Lösungen von Chitosanen in Kontakt bringt und dabei eine Hülle ausformt.

Überraschenderweise wurde gefunden, dass die neuen Nanokapseln einen so geringen Durchmesser aufweisen, dass sie zwischen die Fibrillen von Fasern - sowohl natürlichen wie synthetischen - eingelagert werden können. Im Gegensatz zu Produkten des Standes der Technik, die auf den Fasern haften, können die neuen Nanokapseln daher ihre Wirkung unmittelbar an dem Ort entfalten, wo sie benötigt werden. Sie erweisen sich zudem auch als deutlich wasserresistenter, d.h. werden sowohl wegen ihres geringen Durchmessers als auch ihrer chemischen Zusammensetzung weniger rasch aufgelöst und ausgewaschen. Schließlich erweichen bzw. schmelzen sie im Vergleich erst bei deutlich höheren Temperaturen, was insbesondere für die Flammschutzausrüstung von Textilien von Bedeutung ist.

Ein weiterer Gegenstand der Erfindung betrifft ein Verfahren zur Herstellung von Nanokapseln mit mittleren Durchmessern im Bereich von 10 bis 5000, vorzugsweise 50 bis 1000 und insbesondere 100 bis 500 nm nm, bestehend aus einer Hüllmembran und einer mindestens einen Wirkstoff enthaltenden Matrix, bei dem man
(a) aus Lecithinen bzw. Phospholipiden, Wachskörpern und Wirkstoffen eine Matrix herstellt,
(b) diese mit wässrigen Lösungen von Chitosanen in Kontakt bringt und dabei eine Hülle ausformt.

### Lecithine und Phospholipide

Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Besonders bevorzugt ist der Einsatz von Sojalecithinen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen für die Bildung der Matrix auch Sphingosine bzw. Sphingolipide in Frage. Vorzugsweise werden die Lecithine bzw. Phospholipide in kosmetisch verträglichen, organischen Lösemitteln wie beispielsweise Propylenglycol eingesetzt.

### Wachskörper

Als Wachskörper kommen sowohl natürliche als auch synthetische Wachse in Frage. Typische Beispiele für natürliche Wachse sind Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs). Beispiele für synthetische Wachse sind Petrolatum, Paraffinwachse, Mikrowachse, chemisch modifizierte Wachse (Hartwachse), wie z.B. Montanesterwachse, Sasolwachse, hydrierte Jojobawachse sowie Polyalkylenwachse und Polyethylenglycolwachse. Ebenfalls geeignet sind bei Raumtemperatur feste Fettalkohole vorzugsweise solche mit 16 bis 22 Kohlenstoffatomen, wie insbesondere Cetylalkohol, Stearylalkohol, Cetylstearylalkohol oder Behenylalkohol, Wachsester von Fettsäuren mit Fettalkoholen, welche ebenfalls bei Raumtemperatur fest sind und vorzugsweise in Summe mindestens 20, vorzugsweise mindestens 26 Kohlenstoffatome enthalten sowie vergleichbare andere Fettstoffe, wie beispielsweise Fettether (z.B. Distearylether) oder Ketone (z.B. Stearon). Es empfiehlt sich die Wachskörper oberhalb ihres jeweiligen Schmelzpunktes, also in der Regel bei 50 bis 95, vorzugsweise 60 bis 70 °C einzusetzen.

### Wirkstoffe

Die Auswahl der Wirkstoffe, die in den neuen Nanokapseln eingeschlossen sind, ist an sich unkritisch. Vorzugsweise handelt es sich um Stoffe, die erst durch mechanische Zerstörung der Mikrokapseln freigesetzt werden. In diesen Fällen kommt den Mikrokapseln die Aufgabe zu, den Kontakt zwischen äußerer Umgebung und Wirkstoff und damit eine chemische Reaktion bzw. einen Abbau zu verhindern. Es kann es sein, dass die in der Kapsel eingeschlossenen Stoffe überhaupt nicht freigesetzt werden sollen und ausschließlich dem Zweck dienen, der Zubereitung ein ästhetisches Äußeres zu verleihen; dies trifft beispielsweise vielfach für Farbstoffe zu. Es ist natürlich klar, dass diese Einsatzformen auch nebeneinander bestehen können. Insbesondere ist es möglich, beispielsweise einen Duftstoff für die spätere Freisetzung zusammen mit einem Farbpigment zu verkapseln, welches der Kapsel ein besonderes Aussehen verleiht.

### Wirkstoffe für kosmetische oder pharmazeutische Anwendungen

Typische Beispiele für Wirkstoffe, wie sie im Bereich der kosmetischen und pharmazeutischen Zubereitungen eingesetzt werden sind Tenside, kosmetische Öle, Perlglanzwachse, Stabilisatoren, biogene Wirkstoffe, Vitamine, Deodorantien, Antitranspirantien, Antischuppenmittel, UV-Lichtschutzfaktoren, Antioxidantien, Konservierungsmittel, Insektenrepellentien, Selbstbräuner, Typrosininhibitoren (Depigmentierungsmittel), Parfümöle und Farbstoffe.

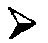 Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid (ether)sulfate, Fettsäureamid(ether)sulfate, Mono- und Dialkylsulfosuccinate, Monound Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart**, **1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

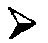 Kosmetische Öle

Als kosmetische Öle kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

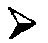 Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

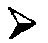 Stabilisatoren

Als Stabilisatoren können Metallsalze von Fettsäuren, wie z.B. Magnesium-, Aluminiumund/oder Zinkstearat bzw. -ricinoleat eingesetzt werden.

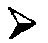 Biogene Wirkstoffe

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Kojisäure, Aminosäuren, Ceramide, Pseudoceramide, essentielle Öle, Pflanzenextrakte und Vitaminkomplexe zu verstehen.

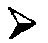 Deodorantien und Antischuppenmittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-N-octylamid oder Salicylsäure-N-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B.entzündungshemmende, hautschützende oder wohlriechende ätherische Öle, synthetische hautschützende Wirkstoffe und/oder öllösliche Parfümöle sein.

Als Antischuppenmittel können Climbazol, Octopirox, Ketokonazol und Zinkpyrethion eingesetzt werden.

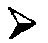 UV-Lichtschutzfaktoren und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen : 3-Benzylidencampher bzw. 3-Benzylidennorcampher und dessen Derivate, z.B. 3-(4-Methylbenzyliden)campher wie in der EP 0693471 B1 beschrieben; 4-Aminobenzoesäurederivate, vorzugsweise 4-(Dimethylamino)benzoesäure-2-ethylhexyl-ester, 4-Dimethylamino)-benzoesäure-2-octylester und 4-(Dimethylamino)benzoesäure-amylester; Ester der Zimtsäure, vorzugsweise 4-Methoxyzimtsäure-2-ethylhexylester, 4-Methoxyzimtsäurepropyl-ester, 4-Methoxyzimtsäureisoamylester 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethyl-hexylester; Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben.

Als wasserlösliche Substanzen kommen in Frage: 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze. Als typische UV-A-Filter kommen insbesondere Derivate des Benzoylmethans in Frage, wie beispielsweise 1-(4'-tert.Butyl-phenyl)-3-(4'-methoxyphenyl)propan-1,3-dion, 4-tert.-Butyl-4'-methoxydibenzoyl-methan (Parsol 1789), 1-Phenyl-3-(4'-isopropylphenyl)-propan-1,3-dion sowie E-naminverbindungen, wie beschrieben in der DE 19712033 A1 (BASF). Die UV-A und UVB-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden als verkapselte Wirkstoffe bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

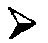 Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insektenrepellentien kommen N,N-Diethyl-m-toluamid, 1,2-Pentandiol oder Ethyl Butylacetylaminopropionate in Frage, als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

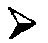 Parfümöle und Farbstoffe

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Wirkstoffe können auch ausschließlich aus ästhetischen Gründen in den Kapseln enthalten und nicht für eine kontrollierte Freigabe vorgesehen sein.

### Wirkstoffe für Anwendungen im Textilbereich

Ein zweiter Einsatzbereich der neuen Nanokapseln besteht in der Ausrüstung von Textilien. Der besondere Vorteil liegt hier darin, dass die Kapseln nicht nur auf den Fasern abgelagert werden, sondern wegen ihres geringen Durchmessern zwischen die Fibrillen gelangen und ihre vorteilhaften Eigenschaften deutlich effektiver zur Geltung bringen können. Typische Beispiele für geeignete Wirkstoffe sind alle Arten von Flammschutzmitteln, also Metalloxide, wie z.B. Magnesiumoxid, Calciumoxid, Aluminiumoxid oder Antimon(III)oxid, organische Halide, wie z.B. Tetrabrombisphenol A oder Decabromodiphenyloxid sowie insbesondere organische Phosphorverbindungen, wie z.B. Triphenylphosphat (TPP), Tricresylphosphat (TCP), Cresyldiphenylphosphat (CDP) oder Tetraphenyldiphosphat. Die Mittel werden vorzugsweise mit Teilchengrößen im Bereich von 0,1 bis 5 mm eingesetzt.

Die Wirkstoffe können dabei in solchen Mengen eingesetzt werden, dass sich in den Nanokapseln ein Gehalt von 0,1 bis 5, vorzugsweise 0,5 bis 3 und insbesondere 1 bis 2 Gew.-% ergibt.

### Tensidische Emulgatoren

In einer weiteren bevorzugten Ausführungsform der vorliegenden Erfindung werden die Lecithine bzw. Phospholipide, Wachskörper und Wirkstoffe gemeinsam mit tensidischen Emulgatoren eingesetzt. Als Emulgatoren kommen beispielsweise nichtionogene Tenside aus mindestens einer der folgenden Gruppen in Frage:
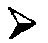 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
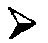 Alkyl- und/oder Alkenyloligoglykoside mit 8 bis 22 Kohlenstoffatomen im Alk(en)ylrest und deren ethoxylierte Analoga;
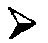 Anlagerungsprodukte von 1 bis 15 Mol Ethylenoxid an Ricinusöl und/oder gehärtetes Ricinusöl;
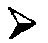 Anlagerungsprodukte von 15 bis 60 Mol Ethylenoxid an Ricinusöl und/oder gehärtetes Ricinusöl;
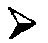 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
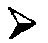 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
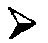 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
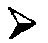 Mono-, Di- und Trialkylphosphate sowie Mono-, Di- und/oder Tri-PEG-alkylphosphate und deren Salze;
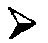 Wollwachsalkohole;
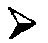 Polysiloxan-Polyalkyl-Polyether-Copolymere bzw. entsprechende Derivate;
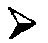 Block-Copolymere z.B. Polyethylenglycol-30 Dipolyhydroxystearate;
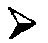 Polymeremulgatoren, z.B. Pemulen-Typen (TR-1,TR-2) von Goodrich;
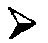 Polyalkylenglycole sowie
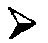 Glycerincarbonat.

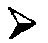 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

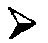 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

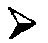 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

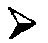 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

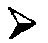 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

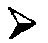 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

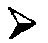 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkyl-sarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Üblicherweise werden die Emulgatoren in Mengen von 1 bis 15, vorzugsweise 2 bis 10 und insbesondere 8 bis 10 Gew.-% - bezogen auf die Summe von Lecithinen/Phospholipiden, Wachsen und Wirkstoffen - eingesetzt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

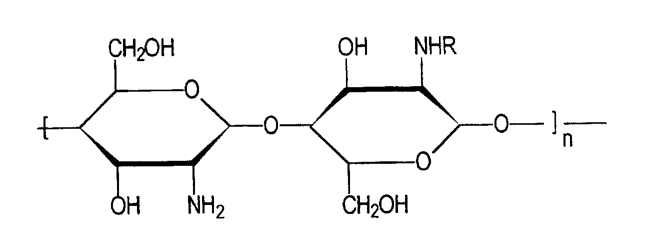

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-232).** Übersichten zu diesem Thema sind auch beispielsweise von B. Gesslein et al. in **HAPPI 27, 57 (1990),** O. Skaugrud in **Drug Cosm.Ind. 148, 24 (1991)** und E. Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt, wobei insbesondere 1 bis 10 Gew.-%ige Lösungen in Betracht kommen.

### Herstellung der Nanokapseln

Zur Herstellung der Nanokapseln stellt man beispielsweise eine 1 bis 10, vorzugsweise 2 bis 5 Gew.-%ige wässrige oder alkoholische Lösung des Lecithins bzw. Phospholipids, vorzugsweise eines Sojalecithins her und vermischt diese unter intensivem Rühren in der Hitze mit dem aufgeschmolzenen Wachs, dem Wirkstoff sowie gegebenenfalls dem Emulgator. Üblicherweise lässt man die Mischung anschließend für etwa 10 bis 30 min ruhen, um die Bildung der Matrix, d.h. der Vesikel, zu begünstigen. Falls gewünscht kann man den Zubereitungen auch organische Co-Lösemittel, beispielsweise kurzkettige Alkohole wie Methanol, Ethanol, Isopropylalkohol, Diole, beispielsweise Ethylenglycol, Diethylenglycol, Propylenglycol, und Ketone wie beispielsweise Aceton zusetzen. Nach der Ausbildung der Matrix bzw. der Vesikel wird die Hülle gebildet. Hierzu bringt man die Zubereitungen mit den wäßrigen Chitosanen in Kontakt. Anschließend empfiehlt es sich, die Mischungen abzukühlen, damit die Wachskörper sich verfestigen können. Gegebenenfalls können danach die organische Co-Solventien und vorhandenes Wasser im Vakuum entfernt werden.

### Gewerbliche Anwendbarkeit

Weitere Gegenstände der Erfindung betreffen sowohl kosmetische und/oder pharmazeutische Zubereitungen als auch Fasern oder daraus hergestellte Textilien bzw. textile Flächengilde, die die erfindungsgemäßen Nanokapseln in Mengen von vorzugsweise 0,1 bis 15 und insbesondere 1 bis 10 Gew.-% enthalten. Weiterhin betroffen ist die Verwendung der Nanokapseln zur Herstellung von kosmetischen bzw. pharmazeutischen Zubereitungen sowie Fasern und/oder daraus hergestellten Textilien oder anderen textilen Flächengebilden, die die Nanokapseln in den oben angegebenen Mengen enthalten.

### Kosmetische und/oder pharmazeutische Zubereitungen

Die erfindungsgemäßen Nanokapseln können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholischen und wässrig/alkoholischen Lösungen, Emulsionen, Wachs/ Fett-Massen, Stiftpräparaten, Pudern oder Salben dienen. Infolge ihres geringen Durchmessers lagern sie sich zwischen die Fibrillen der Keratinfasern ein und können dort nach Freisetzung des Wirkstoffs ihre vorteilhaften Eigenschaften entfalten. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Die entsprechenden Zusatzstoffe sind bereits in den Kapitel Wirkstoffe/tensidische Emulgatoren erläutert worden, so dass auf eine Wiederholung an dieser Stelle verzichtet werden kann.

### Textilien

Die erfindungsgemäßen Nanokapseln können analog zum Einsatz bei natürlichen Keratinfasern auch zur Ausrüstung von Textilien dienen, obschon hier der Schwerpunkt in der Verbesserung des Flammschutzes liegt. Auch hier lagern sich die Nanokapseln direkt zwischen die Faserfilamente ein, was beispielsweise durch Tauchbadimprägnierung und anschließende Trocknung erfolgen kann. In gleicher Weise können die Garne ausgerüstet werden, indem man die Nanokapseln beispielsweise als Bestandteile von Spulölen oder Spinnfaserpräparationen aufbringt. Demzufolge eignen sich die Nanokapseln zur Ausrüstung aller textiler Flächengebilde. In einer besonderen Ausführungsform der vorliegenden Erfindung enthalten die Nanokapseln neben den Flammschutzmitteln noch fettlösliche Farbstoffe, wie beispielsweise Beta-Carotine oder Tocopherole, die dann als Indikatoren dafür dienen können, dass die Kapseln vom Gewebe aufgenommen worden sind.

### Beispiele

**Beispiel 1**. In einem 500-ml-Dreihalskolben mit Rührer und Rückflusskühler wurden bei 60 °C eine Lösung von 30 g Sojalecithin in 150 ml Propylenglycol vorgelegt. In einer zweiten Vorlage wurden 10 g Cetylstearylalkohol (Lanette® O, Cognis Deutschland GmbH) aufgeschmolzen und mit 3 g Sorbitanmonolaurat+20 EO (Eumulgin® SML-20, Cognis Deutschland GmbH) und 5 g Dicaprylylcarbonat (Cetiol® CC, Cognis Deutschland GmbH) versetzt. Die auf diesem Wege hergestellte Mischung wurde bei 60 °C portionsweise zur ersten Vorlage hinzugegeben, die vereinigten Lösungen dann in 300 ml Wasser eingerührt und die nunmehr erhaltene dritte Mischung in einem Ultra-Turrax 10 min intensiv gerührt. Zur Ausbildung der Vesikel ("Matrix") wurde die Mischung dann 15 min sich selbst überlassen und dann mit 75 ml Ethanol verrührt. Auch hierbei wurde die Temperatur bei etwa 60 °C gehalten. Anschließend wurden 200 ml einer 15 Gew.-%igen Lösung von Chitosan Glycolat (Hydagen® CMF, Cognis Deutschland GmbH) versetzt. Dies geschah portionsweise, um eine möglichst vollständige Bildung der Hülle durch Reaktion des Lecithins mit dem Chitosan sicherzustellen. Schließlich wurde die Zubereitung auf 20 °C abgekühlt, wobei sich das Wachs verfestigte, und sowohl das Wasser als auch das Ethanol im Vakuum entfernt. Die so erhaltenen Nanokapseln zeigten einen mittleren Durchmesser von 37 nm.

**Beispiel 2.** Analog Beispiel 1 wurden zunächst 30 g Sojalecithin, 10 g Paraffinwachs, 3 g Cetearylglucoside (and) Cetearylalkohol (Emulgade® PL 68/50) und 2 g Beta-Carotin zu einer Matrix verarbeitet. Diese wurde dann mit 30 g Chitosan Glycolat verkapselt. Die Herstelltemperatur betrug 85 °C, die resultierenden Nanokapseln besaßen einen mittleren Durchmesser von 55 nm.

**Beispiel 3.** Analog Beispiel 1 wurden zunächst 30 g Sojalecithin, 10 g Myristylmyristat (Cetiol® MM, Cognis Deutschland GmbH), 3 g PEG-2 Dipolyhydroxystearate (Dehymuls® PGPH, Cognis Deutschland GmbH) und 1 g Retinol zu einer Matrix verarbeitet. Diese wurde dann mit 30 g Chitosan Glycolat verkapselt. Die Herstelltemperatur betrug 75 °C, die resultierenden Nanokapseln besaßen einen mittleren Durchmesser von 48 nm.

**Beispiel 4.** Analog Beispiel 1 wurden zunächst 30 g Sojalecithin, 10 g Carnaubawachs, 3 g Sorbitanmonostearat+15 EO (Eumulgin® SMS-15, Cognis Deutschland GmbH) und 3 g Tricresylphosphat zu einer Matrix verarbeitet. Diese wurde dann mit 30 g Chitosan Glycolat verkapselt. Die Herstelltemperatur betrug 85 °C, die resultierenden Nanokapseln besaßen einen mittleren Durchmesser von 31 nm.

**Tabelle 1**

| **Beispiele für kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** | - | - | - | - | - | - | 38,0 | 38,0 | 25,0 | - |
| Sodium Laureth Sulfate | | | | | | | | | | |
| **Texapon® SB 3** | - | - | - | - | - | - | - | - | 10,0 | - |
| Disodium Laureth Sulfosuccinate | | | | | | | | | | |
| **Plantacare® 818** | - | - | - | - | - | - | 7,0 | 7,0 | 6,0 | - |
| Coco Glucosides | | | | | | | | | | |
| **Plantacare® PS 10** | - | - | - | - | - | - | - | - | - | 16,0 |
| Sodium Laureth Sulfate (and) Coco Glucosides | | | | | | | | | | |
| **Dehyton® PK 45** | - | - | - | - | - | - | - | - | 10,0 | - |
| Cocamidopropyl Betaine | | | | | | | | | | |
| **Dehyquart® A** | 2,0 | 2,0 | 2,0 | 2,0 | 4,0 | 4,0 | - | - | - | - |
| Cetrimonium Chloride | | | | | | | | | | |
| **Dehyquart L® 80** | 1,2 | 1,2 | 1,2 | 1,2 | 0,6 | 0,6 | - | - | - | - |
| Dicocoylmethylethoxymonium Methosulfate (and) Propylenglycol | | | | | | | | | | |
| **Eumulgin® B2** | 0,8 | 0,8 | - | 0,8 | - | 1,0 | - | - | - | - |
| Ceteareth-20 | | | | | | | | | | |
| **Eumulgin® VL 75** | - | - | 0,8 | - | 0.8 | - | - | - | - | - |
| Lauryl Glucoside (and) Polyglyceryl-2 Polyhydroxystearate (and) Glycerin | | | | | | | | | | |
| **Lanette® O** | 2,5 | 2,5 | 2,5 | 2,5 | 3,0 | 2,5 | - | - | - | - |
| cetearyl Alcohol | | | | | | | | | | |
| **Cutina® GMS** | 0,5 | 0,5 | 0,5 | 0,5 | 0,5 | 1,0 | - | - | - | - |
| Glyceryl Stearate | | | | | | | | | | |
| **Cetiol® HE** | 1,0 | - | - | - | - | - | - | - | 1,0 | |
| PEG-7 Glyceryl Cocoate | | | | | | | | | | |
| **Cetiol® PGL** | - | 1,0 | - | - | 1,0 | - | - | - | - | - |
| Hexyldecanol (and) Hexyldecyl Laurate | | | | | | | | | | |
| **Cetiol® V** | - | - | - | 1,0 | - | - | - | - | - | - |
| Decyl Oleate | | | | | | | | | | |
| **Eutanol® G** | - | - | 1,0 | - | - | 1,0 | - | - | - | - |
| Octyldodecanol | | | | | | | | | | |
| **Nutrilan® Keratin W** | - | - | - | 2,0 | - | - | - | - | - | - |
| Hydrolyzed Keratin | | | | | | | | | | |
| **Lamesoft® LMG** | - | - | - | - | - | - | 3,0 | 2,0 | 4,0 | - |
| Glyceryl Laurate (and) Potassium Cocoyl Hydrolyzed | | | | | | | | | | |
| Collagen | | | | | | | | | | |
| **Euperlan® PK 3000 AM** | - | - | - | - | - | - | - | 3,0 | 5,0 | 5,0 |
| Glycol Distearate (and) Laureth-4 (and) Cocamidopropyl | | | | | | | | | | |
| Betaine | | | | | | | | | | |
| **Generol® 122 N** | - | - | - | - | 1,0 | 1,0 | - | - | - | - |
| Soja Sterol | | | | | | | | | | |
| **Retinol-Nanokapseln gem. Bsp. 3** | 1,0 | 1,0 | 1,0 | 1,0 | 1,0 | 1,0 | 1,0 | 1,0 | 1,0 | 1,0 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Copherol® 1250** | - | - | 0,1 | 0,1 | - | - | - | - | - | - |
| Tocopherol Acetate | | | | | | | | | | |
| **Arlypon® F** | - | - | - | - | - | - | 3,0 | 3,0 | 1,0 | - |
| Laureth-2 | | | | | | | | | | |
| **Sodium Chloride** | - | - | - | - | - | - | - | 1,5 | - | 1,5 |
| (1-4) Haarspülung, (5-6) Haarkur, (7-8) Duschbad, (9) Duschgel, (10) Waschlotion | | | | | | | | | | |

| **Zusammensetzung (INCI)** | **11** | **12** | **13** | **14** | **15** | **16** | **17** | **18** | **19** | **20** |
|---|---|---|---|---|---|---|---|---|---|---|
| **Texapon® NSO** | 20,0 | 20,0 | 12,4 | - | 25,0 | 11,0 | - | - | - | - |
| Sodium Laureth Sulfate | | | | | | | | | | |
| **Texapon® K 14 S** | - | - | - | - | - | - | - | - | 11,0 | 23,0 |
| Sodium Myreth Sulfate | | | | | | | | | | |
| **Texapon® SB 3** | - | - | - | - | - | 7,0 | - | - | - | - |
| Disodium Laureth Sulfosuccinate | | | | | | | | | | |
| **Plantacare® 818** | 5,0 | 5,0 | 4,0 | - | - | - | - | - | 6,0 | 4,0 |
| Coco Glucosides | | | | | | | | | | |
| **Plantacare® 2000** | - | - | - | - | 5,0 | 4,0 | - | - | - | - |
| Decyl Glucoside | | | | | | | | | | |
| **Plantacare® PS 10** | - | - | - | 40,0 | - | - | 16,0 | 17,0 | - | - |
| Sodium Laureth Sulfate (and) Coco Glucosides | | | | | | | | | | |
| **Dehyton® PK 45** | 20,0 | 20,0 | - | - | 8,0 | - | - | - | - | 7,0 |
| Cocamidopropyl Betaine | | | | | | | | | | |
| **Eumulgin® B1** | - | - | - | - | 1,0 | - | - | - | - | - |
| Ceteareth-12 | | | | | | | | | | |
| **Eumulgin® B2** | - | - | - | 1,0 | - | - | - | - | - | - |
| Ceteareth-20 | | | | | | | | | | |
| **Lameform® TGI** | - | - | - | 4,0 | - | - | - | - | - | - |
| Polyglyceryl-3 Isostearate | | | | | | | | | | |
| **Dehymuls® PGPH** | - | - | 1,0 | - | - | - | - | - | - | - |
| Polyglyceryl-2 Dipolyhydroxystearate | | | | | | | | | | |
| **Monomuls® 90-L 12** | - | - | - | - | - | - | - | - | 1,0 | 1,0 |
| Glyceryl Laurate | | | | | | | | | | |
| **Cetiol® HE** | - | 0,2 | - | - | - | - | - | - | - | - |
| PEG-7 Glyceryl Cocoate | | | | | | | | | | |
| **Eutanol® G** | - | - | - | 3,0 | - | - | - | - | - | - |
| Octyldodecanol | | | | | | | | | | |
| **Nutrilan® Keratin W** | - | - | - | - | - | - | - | - | 2,0 | 2,0 |
| Hydrolyzed Keratin | | | | | | | | | | |
| **Nutrilan® I** | 1,0 | - | - | - | - | 2,0 | - | 2,0 | - | - |
| Hydrolyzed Collagen | | | | | | | | | | |
| **Lamesoft® LMG** | - | - | - | - | - | - | - | - | 1,0 | - |
| Glyceryl Laurate (and) Potassium Cocoyl Hydrolyzed | | | | | | | | | | |
| Collagen | | | | | | | | | | |
| **Lamesoft® 156** | - | - | - | - | - | - | - | - | - | 5,0 |
| Hydrogenated Tallow Gyceride (and) Potassium Cocoyl | | | | | | | | | | |
| Hydrolyzed Collagen | | | | | | | | | | |
| **Gluadin® WK** | 1,0 | 1,5 | 4,0 | 1,0 | 3,0 | 1,0 | 2,0 | 2,0 | 2,0 | - |
| Sodium Cocoyl Hydrolyzed Wheat Protein | | | | | | | | | | |
| **Euperlan® PK 3000 AM** | 5,0 | 3,0 | 4,0 | - | - | - | - | 3,0 | 3,0 | - |
| Glycol Distearate (and) Laureth-4 (and) Cocamidopropyl | | | | | | | | | | |
| Betaine | | | | | | | | | | |
| **Arlypon® F** | 2,6 | 1,6 | - | 1,0 | 1,5 | - | - | - | - | - |
| Laureth-2 | | | | | | | | | | |
| **Retinol-Nanokapsein gem. Bsp. 3** | 1,0 | 1,0 | 1,0 | 1,0 | 1,0 | 1,0 | 1,0 | 1,0 | 1,0 | 1,0 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |
| (11-14) Duschbad "Two-in-One), (15-20) Shampoo | | | | | | | | | | |

| **Zusammensetzung (INCI)** | **21** | **22** | **23** | **24** | **25** | **26** | **27** | **28** | **29** | **30** |
|---|---|---|---|---|---|---|---|---|---|---|
| **Texapon® NSO** | - | 30,0 | 30,0 | - | 25,0 | - | - | - | - | - |
| Sodium Laureth Sulfate | | | | | | | | | | |
| **Plantacare® 818** | - | 10,0 | - | - | 20,0 | - | - | - | - | - |
| Coco Glucosides | | | | | | | | | | |
| **Plantacare® PS 10** | 22,0 | - | 5,0 | 22,0 | - | - | - | - | - | - |
| Sodium Laureth Sulfate (and) Coco Glucosides | | | | | | | | | | |
| **Dehyton® PK 45** | 15,0 | 10,0 | 15,0 | 15,0 | 20,0 | - | - | - | - | - |
| Cocamidopropyl Betaine | | | | | | | | | | |
| **Emulgade® SE** | - | - | - | - | - | 5,0 | 5,0 | 4,0 | - | - |
| Glyceryl Sterate (and) Ceteareth 12/20 (and) Cetearyl | | | | | | | | | | |
| Alcohol (and) Cetyl Palmitate | | | | | | | | | | |
| **Eumulgin® B1** | - | - | - | - | - | - | - | 1,0 | - | - |
| Ceteareth-12 | | | | | | | | | | |
| **Lameform® TGI** | - | - | - | - | - | - | - | - | 4,0 | - |
| Polyglyceryl-3 Isostearate | | | | | | | | | | |
| **Dehymuls® PGPH** | - | - | - | - | - | - | - | - | - | 4,0 |
| Polyglyceryl-2 Dipolyhydroxystearate | | | | | | | | | | |
| **Monomuls® 90-O 18** | - | - | - | - | - | - | - | - | 2,0 | - |
| Glyceryl Oleate | | | | | | | | | | |
| **Cetiol® HE** | 2,0 | - | - | 2,0 | 5,0 | - | - | - | - | 2,0 |
| PEG-7 Glyoeryl Cocoate | | | | | | | | | | |
| **Cetiol® OE** | - | - | - | - | - | - | - | - | 5,0 | 6,0 |
| Dicaprylyl Ether | | | | | | | | | | |
| **Cetiol® PGL** | - | - | - | - | - | - | - | 3,0 | 10,0 | 9,0 |
| Hexyldecanol (and) Hexyldecyl Laurate | | | | | | | | | | |
| **Cetiol® SN** | - | - | - | - | - | 3,0 | 3,0 | - | - | - |
| Cetearyl Isononanoate | | | | | | | | | | |
| **Cetiol® V** | - | - | - | - | - | 3,0 | 3,0 | - | - | - |
| Decyl Oleate | | | | | | | | | | |
| **Myritol® 318** | - | - | - | - | - | - | - | 3,0 | 5,0 | 5,0 |
| Coco Caprylate Caprate | | | | | | | | | | |
| **Bees Wax** | - | - | - | - | - | - | - | - | 7,0 | 5,0 |
| **Nutrilan® Elastin E20** | - | - | - | - | - | 2,0 | - | - | - | - |
| Hydrolyzed Elastin | | | | | | | | | | |
| **Nutrilan® I-50** | - | - | - | - | 2,0 | - | 2,0 | - | - | - |
| Hydrolyzed Collagen | | | | | | | | | | |
| **Gluadin® AGP** | 0,5 | 0,5 | 0,5 | - | - | - | - | 0,5 | - | - |
| Hydrolyzed Wheat Gluten | | | | | | | | | | |
| **Gluadin® WK** | 2,0 | 2,0 | 2,0 | 2,0 | 5,0 | - | - | - | 0,5 | 0,5 |
| Sodium Cocoyl Hydrolyzed Wheat Protein | | | | | | | | | | |
| **Euperlan® PK 3000 AM** | 5,0 | - | - | 5,0 | - | - | - | - | - | - |
| Glycol Distearate (and) Laureth-4 (and) Cocamidopropyl | | | | | | | | | | |
| Betaine | | | | | | | | | | |
| **Arlypon® F** | - | - | - | - | - | - | - | - | - | - |
| Laureth-2 | | | | | | | | | | |
| **Retinol-Nanokapseln gem. Bsp. 3** | 1,0 | 1,0 | 1,0 | 1,0 | 1,0 | 1,0 | 1,0 | 1,0 | 1,0 | 1,0 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Magnesium Sulfate Hepta Hydrate** | - | - | - | - | - | - | - | - | 1,0 | 1,0 |
| **Glycerin** (86 Gew.-%ig) | - | - | - | - | - | 3,0 | 3,0 | 5,0 | 5,0 | 3,0 |
| (21-25) Schaumbad, (26) Softcreme, (27, 28) Feuchtigkeitsemulsion, (29, 30) Nachtcreme | | | | | | | | | | |

| **Zusammensetzung (INCI)** | **31** | **32** | **33** | **34** | **35** | **36** | **37** | **38** | **39** | **40** |
|---|---|---|---|---|---|---|---|---|---|---|
| **Dehymuls® PGPH** | 4,0 | 3,0 | - | 5,0 | - | - | - | - | - | - |
| Polyglyceryl-2 Dipolyhydroxystearate | | | | | | | | | | |
| **Lameform® TGI** | 2,0 | 1,0 | - | - | - | - | - | - | - | - |
| Polyglyceryl-3 Diisostearate | | | | | | | | | | |
| **Emulgade® PL 68/50** | - | - | - | - | 4,0 | - | - | - | 3,0 | - |
| Cetearyl Glucoside (and) Cetearyl Alcohol | | | | | | | | | | |
| **Eumulgin®B2** | - | - | - | - | - | - | - | 2,0 | - | - |
| Ceteareth-20 | | | | | | | | | | |
| **Tegocare® PS** | - | - | 3,0 | - | - | - | 4,0 | - | - | - |
| Polyglyceryl-3 Methylglucose Distearate | | | | | | | | | | |
| **Eumulgin VL 75** | - | - | - | - | - | 3,5 | - | - | 2,5 | - |
| Polyglyceryl-2 Dipolyhydroxystearate (and) Lauryl Gluco- | | | | | | | | | | |
| side (and) Glycerin | | | | | | | | | | |
| **Bees Wax** | 3,0 | 2,0 | 5,0 | 2,0 | - | - | - | - | - | - |
| **Cutina® GMS** | - | - | - | - | - | 2,0 | 4,0 | - | - | 4,0 |
| Glyceryl Stearate | | | | | | | | | | |
| **Lanette® O** | - | - | 2,0 | - | 2,0 | 4,0 | 2,0 | 4,0 | 4,0 | 1,0 |
| Cetearyl Alcohol | | | | | | | | | | |
| **Antaron® V 216** | - | - | - | - | - | 3,0 | - | - | - | 2,0 |
| PVP / Hexadecene Copolymer | | | | | | | | | | |
| **Myritol® 818** | 5,0 | - | 10,0 | - | 8,0 | 6,0 | 6,0 | - | 5,0 | 5,0 |
| Cocoglycerides | | | | | | | | | | |
| **Finsolv® TN** | - | 6,0 | - | 2,0 | - | - | 3,0 | - | - | 2,0 |
| C12/15 Alkyl Benzoate | | | | | | | | | | |
| **Cetiol® J 600** | 7,0 | 4,0 | 3,0 | 5,0 | 4,0 | 3,0 | 3,0 | - | 5,0 | 4,0 |
| Oleyl Erucate | | | | | | | | | | |
| **Cetiol® OE** | 3,0 | - | 6,0 | 8,0 | 6,0 | 5,0 | 4,0 | 3,0 | 4,0 | 6,0 |
| Dicaprylyl Ether | | | | | | | | | | |
| **Mineral Oil** | - | 4,0 | - | 4,0 | - | 2,0 | - | 1,0 | - | - |
| **Cetiol® PGL** | - | 7,0 | 3,0 | 7,0 | 4,0 | - | - | - | 1,0 | - |
| Hexadecanol (and) Hexyldecyl Laurate | | | | | | | | | | |
| **Bisabolol** | 1,2 | 1,2 | 1,2 | 1,2 | 1,2 | 1,2 | 1,2 | 1,2 | 1,2 | 1,2 |
| **Retinol-Nanokapseln gem. Bsp. 3** | 1,0 | 1,0 | 1,0 | 1,0 | 1,0 | 1,0 | 1,0 | 1,0 | 1,0 | 1,0 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Copherol® F 1300** | 0,5 | 1,0 | 1,0 | 2,0 | 1,0 | 1,0 | 1,0 | 2,0 | 0,5 | 2,0 |
| Tocopherol / Tocopheyl Acetate | | | | | | | | | | |
| **Neo Heliopan® Hydro** | 3,0 | - | - | 3,0 | - | - | 2,0 | - | 2,0 | - |
| Sodium Phenylbenzimidazole Sulfonate | | | | | | | | | | |
| **Neo Heliopan® 303** | - | 5,0 | - | - | - | 4,0 | 5,0 | - | - | 10,0 |
| Octocrylene | | | | | | | | | | |
| **Neo Heliopan® BB** | 1,5 | - | - | 2,0 | 1,5 | - | - | - | 2,0 | - |
| Benzophenone-3 | | | | | | | | | | |
| **Neo Heliopan® E 1000** | 5,0 | - | 4,0 | - | 2,0 | 2,0 | 4,0 | 10,0 | - | - |
| Isoamyl p-Methoxycinnamate | | | | | | | | | | |
| **Neo Heliopan® AV** | 4,0 | - | 4,0 | 3,0 | 2,0 | 3,0 | 4,0 | - | 10,0 | 2,0 |
| Octyl Methoxycinnamate | | | | | | | | | | |
| **Uvinul® T 150** | 2,0 | 4,0 | 3,0 | 1,0 | 1,0 | 1,0 | 4,0 | 3,0 | 3,0 | 3,0 |
| Octyl Triazone | | | | | | | | | | |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (31) W/O-Sonnenschutzcreme, (32-34) W/O-Sonnenschutzlotion, (35, 38, 40) O/W-Sonnenschutzlotion (36, 37, 39) O/W-Sonnenschutzcreme | | | | | | | | | | |

## Patentansprüche

1. Nanokapseln mit mittleren Durchmessern im Bereich von 10 bis 5000 nm, bestehend aus einer Hüllmembran und einer mindestens einen Wirkstoff enthaltenden Matrix, dadurch erhältlich, dass man
(a) aus Lecithinen bzw. Phospholipiden, Wachskörpern und Wirkstoffen eine Matrix herstellt,
(b) diese mit wässrigen Lösungen von Chitosanen in Kontakt bringt und dabei eine Hülle ausformt.

2. Verfahren zur Herstellung von Nanokapseln mit mittleren Durchmessern im Bereich von 10 bis 5000 nm, bestehend aus einer Hüllmembran und einer mindestens einen Wirkstoff enthaltenden Matrix, bei dem man
(a) aus Lecithinen bzw. Phospholipiden, Wachskörpern und Wirkstoffen eine Matrix herstellt,
(b) diese mit wässrigen Lösungen von Chitosanen in Kontakt bringt und dabei eine Hülle ausformt.

3. Verfahren nach Anspruch 2, **dadurch gekennzeichnet, dass** man als Wachskörper natürliche oder synthetische Wachse einsetzt.

4. Verfahren nach den Ansprüchen 2 und/oder 3, **dadurch gekennzeichnet, dass** man Wachskörper einsetzt, die ausgewählt sind aus der Gruppe, die gebildet wird von Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachsen, Mikrowachsen, Montanesterwachsen, Sasolwachsen, hydrierten Jojobawachen, Polyalkylenwachsen, Polyethylenglycolwachsen sowie bei Raumtemperatur festen Fettalkoholen, Wachsestern, Fettethern und Fettketonen.

5. Verfahren nach mindestens einem der Ansprüche 2 bis 4, **dadurch gekennzeichnet, dass** man kosmetische und/oder pharmazeutische Wirkstoffe einsetzt, die ausgewählt sind aus der Gruppe, die gebildet wird von kosmetischen Ölen, Perlglanzwachsen, Stabilisatoren, biogenen Wirkstoffen, Vitaminen, Deodorantien, Antitranspirantien, Antischuppenmitteln, UV-Lichtschutzfaktoren, Antioxidantien, Konservierungsmitteln, Insektenrepellentien, Selbstbräunern, Typrosininhibitoren, Parfümölen und Farbstoffen.

6. Verfahren nach mindestens einem der Ansprüche 2 bis 5, **dadurch gekennzeichnet, dass** man Flammschutz-Wirkstoffe einsetzt, die ausgewählt sind aus der Gruppe, die gebildet wird von Metalloxiden, organischen Haliden und organischen Phosphorverbindungen.

7. Verfahren nach mindestens einem der Ansprüche 2 bis 6, **dadurch gekennzeichnet, dass** man die Wirkstoffe in solchen Mengen einsetzt, dass sich in den Nanokapseln eine Menge von 0,1 bis 5 Gew.-% ergibt.

8. Verfahren nach mindestens einem der Ansprüche 2 bis 7, **dadurch gekennzeichnet, dass** man die Matrix weiterhin in Gegenwart von tensidischen Emulgatoren herstellt.

9. Verfahren nach Anspruch 8, **dadurch gekennzeichnet, dass** man anionische, nichtionische, amphotere und/oder kationische Emulgatoren einsetzt.

10. Verfahren nach den Ansprüchen 8 und/oder 9, **dadurch gekennzeichnet, dass** man die tensidischen Emulgatoren in Mengen von 1 bis 15 Gew.-% - bezogen auf die Summe aus Lecithine/Phospholipiden, Wachsen und Wirkstoffen - einsetzt.

11. Verfahren nach mindestens einem der Ansprüche 1 bis 10, **dadurch gekennzeichnet, dass** man Chitosane einsetzt, die ein durchschnittliches Molekulargewicht von 10.000 bis 500.000 aufweisen.

12. Verfahren nach mindestens einem der Ansprüche 2 bis 11, **dadurch gekennzeichnet, dass** man wässrige oder alkoholische Lösungen des Lecithins und/oder Phospholipids herstellt und diese unter intensivem Rühren in der Hitze mit dem aufgeschmolzenen Wachs, dem Wirkstoff sowie gegebenenfalls dem Emulgator sowie organischen Co-Solventien vermischt, die Zubereitungen 10 bis 30 min ruhen lässt, die gebildete Matrix zur Bildung der Hülle mit den wässrigen Chitosanen in Kontakt bringt, zur Verfestigung der Wachse abkühlt und gegebenenfalls Wasser und/oder organische Co-Solventien von den Nanokapseln im Vakuum abtrennt.

13. Kosmetische und/oder pharmazeutische Zubereitungen, enthaltend Nanokapseln gemäß Anspruch 1.

14. Fasern und/oder daraus hergestellte Textilien bzw. textile Flächengebilde, enthaltend Nanokapseln gemäß Anspruch 1.

15. Verwendung von Nanokapseln nach Anspruch 1 zur Herstellung von kosmetischen und/oder pharmazeutischen Zubereitungen.

16. Verwendung von Nanokapseln nach Anspruch 1, zur Herstellung von Fasern und/oder daraus hergestellten Textilien bzw. anderen textilen Flächengebilden.

## Claims

1. Nanocapsules with mean diameters of 10 to 5,000 nm consisting of a membrane and a matrix containing at least one active component, obtainable by
(a) preparing a matrix from lecithins or phospholipids, waxes and active components,
(b) contacting the matrix with aqueous solutions of chitosans to form a membrane.

2. A process for the production of nanocapsules with mean diameters of 10 to 5,000 nm consisting of a membrane and a matrix containing at least one active component, in which
(a) a matrix is prepared from lecithins or phospholipids, waxes and active components,
(b) the matrix is contacted with aqueous solutions of chitosans to form a membrane.

3. A process as claimed in claim 2, **characterized in that** natural or synthetic waxes are used as the wax.

4. A process as claimed in claims 2 and/or 3, **characterized in that** the wax used is selected from the group consisting of candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes, montan ester waxes, sasol waxes, hydrogenated jojoba waxes, polyalkylene waxes, polyethylene glycol waxes and fatty alcohols solid at room temperature, wax esters, fatty ethers and fatty ketones.

5. A process as claimed in at least one of claims 2 to 4, **characterized in that** cosmetic and/or pharmaceutical active components selected from the group consisting of cosmetic oils, pearlizing waxes, stabilizers, biogenic agents, vitamins, deodorants, antiperspirants, antidandruff agents, UV protection factors, antioxidants, preservatives, insect repellents, self-tanning agents, tyrosine inhibitors, perfume oils and dyes are used.

6. A process as claimed in at least one of claims 2 to 5, **characterized in that** flame retardants selected from the group consisting of metal oxides, organic halides and organic phosphorus compounds are used.

7. A process as claimed in at least one of claims 2 to 6, **characterized in that** the active components are used in such quantities that they are present in the nanocapsules in a quantity of 0.1 to 5% by weight.

8. A process as claimed in at least one of claims 2 to 7, **characterized in that** the matrix is prepared in the additional presence of surface-active emulsifiers.

9. A process as claimed in claim 8, **characterized in that** anionic, nonionic, amphoteric and/or cationic emulsifiers are used.

10. A process as claimed in claims 8 and/or 9, **characterized in that** the surface-active emulsifiers are used in quantities of 1 to 15% by weight, based on the sum of lecithins/phospholipids, waxes and active components.

11. A process as claimed in at least one of claims 1 to 10, **characterized in that** chitosans with an average molecular weight of 10,000 to 500,000 are used.

12. A process as claimed in at least one of claims 2 to 11, **characterized in that** aqueous or alcoholic solutions of the lecithin and/or phospholipid are prepared and intensively mixed while heating with the molten wax, the active component and optionally the emulsifier and organic co-solvents, the preparations are left standing for 10 to 30 mins., the matrix formed is contacted with the aqueous chitosans to form the membrane, cooled to solidify the waxes and water and/or organic co-solvents are optionally removed in vacuo from the nanocapsules.

13. Cosmetic and/or pharmaceutical preparations containing the nanocapsules claimed in claim 1.

14. Fibres and/or textiles or fabrics produced therefrom containing the nanocapsules claimed in claim 1.

15. The use of the nanocapsules claimed in claim 1 for the production of cosmetic and/or pharmaceutical preparations.

16. The use of the nanocapsules claimed in claim 1 for the production of fibres and/or textiles or other fabrics formed therefrom.

## Revendications

1. Nanocapsules ayant des diamètres moyens allant de 10 à 5000 nm, constituées d'une membrane enveloppe et d'une matrice contenant au moins une substance active, que l'on peut obtenir
(a) en produisant une matrice à partir de lécithines ou de phospholipides, de corps cireux et de substances actives,
(b) en mettant cette matrice en contact avec des solutions aqueuses de chitosanes et en formant ainsi une enveloppe.

2. Procédé de production de nanocapsules ayant des diamètres moyens allant de 10 à 5000 nm, constituées d'une membrane enveloppe et d'une matrice contenant au moins une substance active, selon lequel
(a) on produit une matrice à partir de lécithines ou de phospholipides, de corps cireux et de substances actives,
(b) on met cette matrice en contact avec des solutions aqueuses de chitosanes et l'on forme ainsi une enveloppe.

3. Procédé selon la revendication 2,
**caractérisé en ce qu'**
on utilise des cires naturelles ou synthétiques comme corps cireux.

4. Procédé selon les revendications 2 et/ou 3,
**caractérisé en ce qu'**
on utilise des corps cireux qui sont choisis dans le groupe formé par la cire de candelilla, la cire de carnauba, la cire du Japon, la cire d'espartogras, la cire subérique, la cire de guaruma, la cire d'huile de germe de riz, la cire de canne à sucre, la cire d'ouricury, la cire de lignite, la cire d'abeilles, la cire de gomme-laque, le spermaceti, la lanoline (cire de laine), la cire de croupion, la cérésine, l'ozokérite (cire fossile), le petrolatum, les cires paraffiniques, les microcires, les cires d'esters de lignite, les cires de sasol, les cires de jojoba hydrogénées, les cires de polyalkylène, les cires de polyéthylèneglycol ainsi que les alcools gras, les esters de cires, les éthers gras et les cétones grasses solides à la température ambiante.

5. Procédé selon au moins une des revendications 2 à 4,
**caractérisé en ce qu'**
on utilise des substances actives cosmétiques et/ou pharmaceutiques choisies dans le groupe formé par les huiles cosmétiques, les cires nacrantes, les stabilisateurs, les substances actives biogènes, les vitamines, les déodorants, les anti-transpirants, les agents anti-pelliculaires, les facteurs de protection contre la lumière UV, les antioxydants, les agents de conservation, les agents répulsifs d'insectes, les agents d'auto-bronzage, les inhibiteurs de tyrosine, les huiles parfumées et les colorants.

6. Procédé selon au moins une des revendications 2 à 5,
**caractérisé en ce qu'**
on utilise des substances actives ignifuges choisies dans le groupe formé par les oxydes métalliques, les halogénures organiques et les composés phosphorés organiques.

7. Procédé selon au moins une des revendications 2 à 6,
**caractérisé en ce qu'**
on utilise les substances actives en quantités telles qu'elles sont contenues dans les nanocapsules en quantité de 0,1à 5 % en poids.

8. Procédé selon au moins une des revendications 2 à 7,
**caractérisé en ce qu'**
on produit en outre la matrice en présence d'émulsifiants tensioactifs.

9. Procédé selon la revendication 8,
**caractérisé en ce qu'**
on utilise des émulsifiants anioniques, non ioniques, amphotères et/ou cationiques.

10. Procédé selon les revendications 8 et/ou 9,
**caractérisé en ce qu'**
on utilise les émulsifiants tensioactifs en quantités de 1 à 15 % en poids - par rapport à la somme des lécithines/phospholipides, des cires et des substances actives.

11. Procédé selon au moins une des revendications 1 à 10,
**caractérisé en ce qu'**
on utilise des chitosanes qui présentent un poids moléculaire moyen de 10 000 à 500 000.

12. Procédé selon au moins une des revendications 2 à 11,
**caractérisé en ce qu'**
on produit des solutions aqueuses ou alcooliques de lécithine et/ou de phospholipide et on les mélange, sous agitation intensive à chaud, avec la cire fondue, la substance active ainsi que, le cas échéant, l'émulsifiant et des co-solvants organiques, on laisse reposer les préparations pendant 10 à 30 minutes, on met la matrice formée en contact avec les chitosanes aqueux pour la formation de l'enveloppe, on refroidit pour solidifier les cires et, le cas échéant on sépare l'eau et/ou les co-solvants organiques des nanocapsules sous vide.

13. Préparations cosmétiques et/ou pharmaceutiques qui contiennent des nanocapsules selon la revendication 1.

14. Fibres et/ou textiles ou articles textiles en nappe fabriqués à partir de celles-ci contenant des nanocapsules selon la revendication 1.

15. Utilisation de nanocapsules selon la revendication 1, pour la production de préparations cosmétiques et/ou pharmaceutiques.

16. Utilisation de nanocapsules selon la revendication 1, pour la production de fibres et/ ou de textiles ou d'autres articles textiles en nappe fabriqués à partir de celles-ci.
